(19) Europäisches Patentamt
European Patent Office
Office européen des brevets

(11) EP 0 794 168 B1

(12) EUROPÄISCHE PATENTSCHRIFT

(45) Veröffentlichungstag und Bekanntmachung des Hinweises auf die Patenterteilung:
**05.06.2002 Patentblatt 2002/23**

(51) Int Cl.[7]: **C07C 68/00**, C08G 64/30, C07C 69/96

(21) Anmeldenummer: **97102966.5**

(22) Anmeldetag: **24.02.1997**

# (54) Verfahren zur Herstellung von Diarylcarbonaten und den daraus erhältlichen Polycarbonaten

Process for the preparation of diarylcarbonates and from these obtainable polycarbonates

Procédé de préparation de carbonates de diaryle et des polycarbonates obtenus à partir de ceux-ci

(84) Benannte Vertragsstaaten:
**BE DE ES FR GB IT NL**

(30) Priorität: **08.03.1996 DE 19609057**

(43) Veröffentlichungstag der Anmeldung:
**10.09.1997 Patentblatt 1997/37**

(73) Patentinhaber: **BAYER AG**
**51368 Leverkusen (DE)**

(72) Erfinder:
- **Zaby, Gottfried, Dr.**
  **51375 Leverkussen (DE)**
- **Buysch, Hans-Josef, Dr.**
  **47809 Krefeld (DE)**
- **Kühling, Steffen, Dr.**
  **40670 Meerbusch (DE)**
- **Hesse, Carsten, Dr.**
  **47800 Krefeld (DE)**
- **Rechner, Johann, Dr.**
  **47906 Kempen (DE)**

(56) Entgegenhaltungen:
**EP-A- 0 654 461** **LU-A- 88 569**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

EP 0 794 168 B1

## Beschreibung

Gegenstand der vorliegenden Erfindung ist ein stofflich geschlossenes und energetisch gekoppeltes, phosgenfreies Gesamtverfahren zur Herstellung von Diarylcarbonaten und von lösungsmittelfreien Polycarbonaten, ausgehend von Bisphenolen und Diarylcarbonaten, das dadurch gekennzeichnet ist, daß man das Monophenol, das bei der Umesterung zum Oligo-/Polycarbonat freigesetzt wird, wiederum zur phosgenfreien Herstellung von Diarylcarbonat durch oxidative Carbonylierung des entsprechenden Monophenols verwendet.

Es ist bekannt, aromatische Carbonate durch oxidative Umsetzung einer aromatischen Hydroxyverbindung mit Kohlenmonoxid in Gegenwart eines Edelmetall-Katalysators herzustellen (DE-OS 2 738 437, JP-01 165 551, WO 93/03000, EP 583 935, EP 583 937 und EP 583 938). Als Edelmetall wird bevorzugt Palladium eingesetzt. Zusätzlich können ein Cokatalysator (z.B. Mangan- oder Kobaltsalze), eine Base, ein quaternäres Salz, verschiedene Chinone bzw. Hydrochinone und Trockenmittel eingesetzt werden. Dabei kann in einem Lösungsmittel, bevorzugt Methylenchlorid, gearbeitet werden.

Bei der Umsetzung aromatischer Hydroxyverbindungen mit Kohlenmonoxid und Sauerstoff wird pro Mol gebildeten organischen Carbonats ein Mol Wasser frei, welches den Fortgang der Reaktion behindert. Die Verwendung von Molsieb zur Absorption des Reaktionswassers, etwa gemäß DE-OS 27 38 437, macht eine technische Nutzung des Verfahrens unattraktiv, da für eine effektive Abtrennung des Wassers aus der Flüssigphase große Mengen Molsieb (100 - 500 % Überschuß) benötigt werden, die unter hohem technischen Aufwand regeneriert werden müssen. Die nach diesem Verfahren erzielbaren Raum-Zeit-Ausbeuten sind für eine technische Anwendung zu gering. Eine großtechnisch kontinuierlich durchführbare Reaktionsführung wird nicht offenbart.

In JP-04 257 546 wird ein Verfahren beschrieben, bei dem organische Carbonate durch oxidative Umsetzung einer aromatischen Hydroxyverbindung mit Kohlenmonoxid in Gegenwart eines Edelmetall-Katalysators und eines quartären Salzes durch kontinuierliches Einspeisen in eine Destillationskolonne bei 150-205°C und 30-50 bar hergestellt werden. Das Reaktionswasser wird kontinuierlich abdestilliert. Nachteilig bei diesem Verfahren ist, daß man zur Entfernung des Reaktionswassers in einer Destillationskolonne arbeiten muß, die aufgrund ihrer Konstruktion nur kurze Verweilzeiten ermöglicht. Die mit diesem Verfahren realisierbaren Raum-Zeit-Ausbeuten sind dem zufolge mit nur 17,8 g/l·h sehr niedrig. Mit der Reaktionsführung in einer Destillationskolonne ist die Verwendung großer Mengen Halogenide bei hohen Temperaturen (150 - 205°C) verbunden. Dies führt zu großen Korrosionsproblemen, die zusätzlich einen hohen apparativen Aufwand bedingen, Dem Fachmann ist außerdem bekannt, daß unter den angegebenen Reaktionsbedingungen das als quaternäres Salz bevorzugt eingesetzte Jodid nicht stabil ist und in erheblichem Maße zu Jod oxidiert wird. Dies führt zu großen Verlusten des quaternären Salzes und zur Bildung von Nebenprodukten, was die Selektivität und damit die Wirtschaftlichkeit dieses Verfahrens stark beeinträchtigt. Bei diesen hohen Temperaturen und Drücken ist außerdem mit einer raschen Desaktivierung des homogenen Katalysatorsystems, verursacht durch die Halogenverluste und das Teilchenwachstum des Palladiums, zu rechnen, so daß eine wirtschaftliche Nutzung dieses Verfahrens nicht möglich ist.

Alle nach dem Stand der Technik bekannten Verfahren werden weiterhin mit frischer, nicht recyclisierter Monohydroxyverbindung betrieben. Eine stoffliche Kopplung mit einer Schmelzepolycarbonatanlage ist bisher unbekannt und schien aus den Offenbarungen des Standes der Technik nicht möglich zu sein. Weiterhin ist die vollständig phosgenfreie Herstellung aromatischer Polycarbonate durch Umesterung von Diarylcarbonaten aus der oxidativen Carbonylierung aromatischer Hydroxyverbindungen mit Bisphenolen bisher nicht beschrieben. Es bestand daher die Aufgabe, ein Verfahren zu finden, das es erlaubt, die Herstellung von Diarylcarbonaten durch oxidative Carbonylierung mit hoher Raum-Zeit-Ausbeute und mit stofflicher Kopplung an eine Schmelzepolycarbonatanlage durchzuführen.

Überraschend wurde nun gefunden, daß die oxidative Carbonylierung der aromatischen Monohydroxyverbindung mit hohen Raum-Zeit-Ausbeuten und einer Selektivität, bezogen auf die Monohydroxyverbindung, von größer 99 % auch dann gelingt, wenn als Monohydroxyverbindung der Rückstrom aus der Schmelzepolycarbonatanlage verwendet wird. Dies war überraschend, da die rückgeführte Monohydroxyverbindung im Vergleich zur frischen Monohydroxyverbindung verunreinigt sein kann und solche Verunreinigungen sowohl die Selektivität als auch die Reaktionsgeschwindigkeit der oxidativen Carbonylierung stark beeinträchtigen können und eine rasche Desaktivierung des Katalysatorystems zu befürchten war.

Dabei ergibt sich ein Gesamtverfahren von Diarylcarbonaten und den daraus durch Schmelzkondensation erhältlichen Polycarbonaten.

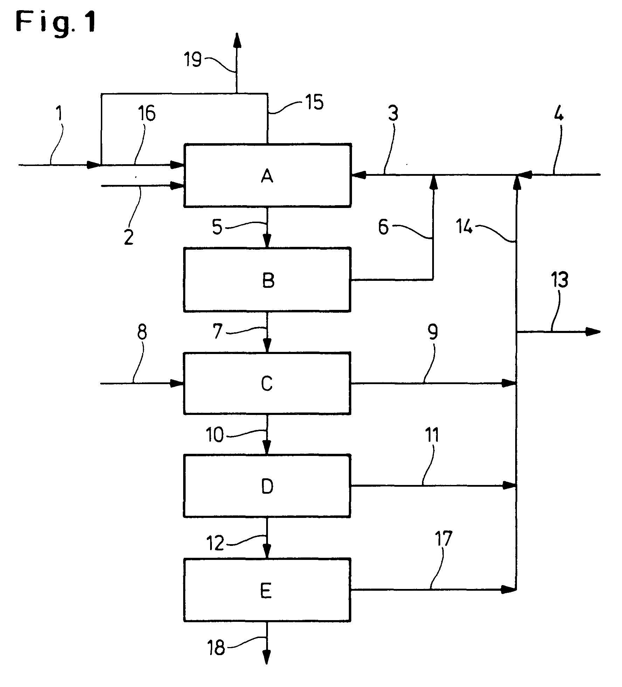

Das erfindungsgemäße Gesamtverfahren ist flexibel, einfach in der Betriebsweise und liefert Produkte in hoher Reinheit, die für den Gesamtprozeß außerordentlich wichtig sind und umfaßt somit folgende Prozeßschritte (vgl. Figur 1):

1. Herstellung des Diarylcarbonats durch oxidative Carbonylierung der zugrundeliegenden aromatischen Hydroxyverbindung,

2. Abtrennung aus dem Herstellungsgemisch und Reinigung des Diarylcarbonats,

3. Umesterung des Diarylcarbonats mit einer aromatischen Dihydroxyverbindung zum Oligo-/Polycarbonat und Freisetzung der aromatischen Monohydroxyverbindung,

4. Isolierung bzw. Trennung des Polycarbonats und der aromatischen Monohydroxyverbindung,

5. Rückführung der aromatischen Monohydroxyverbindung ohne weitere Reinigung in die Herstellung des Diarylcarbonats.

Die Erfindung betrifft demnach ein Verfahren zur Herstellung eines Diarylcarbonats der Formel (I)

$$R^1\text{-O-CO-O-}R^1 \quad (I)$$

durch Umsetzung der zugrundeliegenden aromatischen Hydroxyverbindung der Formel (II)

$$R^1\text{-OH} \quad (II),$$

wobei in den Formeln

$R^1$ Phenyl, Methyl-phenyl, Ethyl-phenyl oder Chlor-phenyl bedeutet,

mit Kohlenmonoxid und Sauerstoff in Gegenwart eines Platinmetall-Katalysators, eines Cokatalysators, eines quaternären Salzes und einer Base, das dadurch gekennzeichnet ist, daß als aromatische Hydroxyverbindungen diejenige eingesetzt wird, die bei der Schmelzpolykondensation von (I) mit einem Bisphenol der Formel

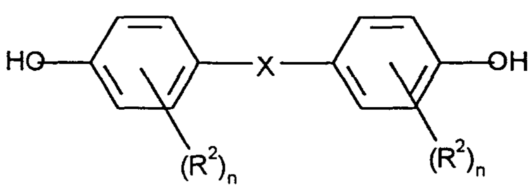
(III),

worin

X eine Einfachbindung, -S-, $-SO_2-$, -CO-, -O-, $C_1$-$C_8$-Alkyliden oder $C_5$-$C_9$-Cycloalkyliden,

$R^2$ Methyl, Cl oder Br und

n eine der Zahlen 0, 1 oder 2 bedeuten,

herausgespalten wird.

Die Erfindung betrifft weiterhin ein Verfahren zur Herstellung von Polycarbonaten durch Schmelzpolykondensation eines Diarylcarbonats mit einem Bisphenol unter Herausspalten der dem Diarylcarbonat zugrundeliegenden aromatischen Hydroxyverbindung, das gekennzeichnet ist durch die Schritte

a) Herstellung eines Diarylcarbonats der Formel

$$R^1\text{-O-CO-O-}R^1 \quad (I)$$

durch oxydative Carbonylierung der zugrundeliegenden aromatischen Hydroxyverbindung der Formel

$R^1$-OH (II),

wobei in den Formeln

$R^1$ Phenyl, Methyl-phenyl, Ethyl-phenyl oder Chlor-phenyl bedeutet,

mit Kohlenmonoxid und Sauerstoff in Gegenwart eines Platinmetall-Katalysators, eines Cokatalysators, eines quaternären Salzes und einer Base,

b) Abtrennung und Reinigung des Diarylcarbonats,

c) Umesterung des Diarylcarbonats mit einem Bisphenol der Formel

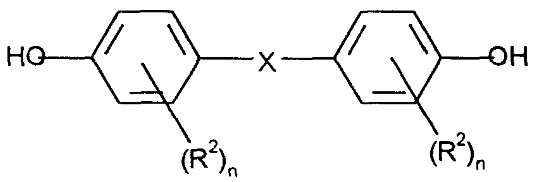

(III),

worin

X eine Einfachbindung, -S-, $-SO_2-$, -CO-, -O-, $C_1$-$C_8$-Alkyliden oder $C_5$-$C_9$-Cycloalkyliden,

$R^2$ Methyl, Cl oder Br und

n eine der Zahlen 0, 1 oder 2 bedeuten,

in einer Schmelzpolykondensation unter Bildung des korrespondierenden Oligo- oder Polycarbonats und Herausspalten der aromatischen Hydroxyverbindung (II),

d) Abtrenung der aromatischen Hydroxyverbindung (II) vom Oligo- bzw. Polycarbonat und

e) Rückführung von (II) in den Schritt a) ohne weitere Reinigung.

Erfindungsgemäß einsetzbare Bisphenole sind beispielsweise: 4,4'-Dihydroxydiphenyl, 4,4'-Dihydroxydiphenylsulfid, 2,2-Bis-(4-hydroxyphenyl)-propan (Bisphenol A), 2,2-Bis-(3,5-dimethyl-4-hydroxyphenyl)-propan, 2,2-Bis-(3,5-dichlor-4-hydroxyphenyl)-propan, 2,2-Bis-(3,5-dibrom-4-hydroxyphenyl)-propan, 1,1-Bis-(4-hydroxyphenyl)-cyclohexan, 1,1-Bis-(4-hydroxyphenyl)-3,3,5-trimethylcyclohexan (mit $C_9$-Cycloalkyliden), Bis-(4-hydroxyphenyl)-sulfon und 4,4'-Dihydroxy-benzophenon.

Bevorzugte Bisphenole sind solche der Formel

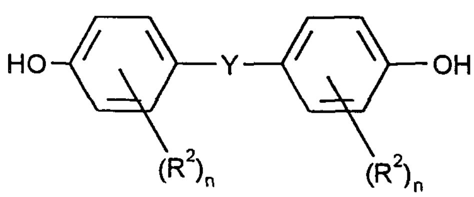

(IV),

in der

Y eine Einfachbindung, -O-, $C_1$-$C_5$-Alkyliden oder $C_6$-$C_9$-Cycloalkyliden bedeuten und

$R^2$ und n die obige Bedeutung haben.

Besonders bevorzugte Bisphenole aus den vorstehend genannten sind 2,2-Bis-(4-hydroxyphenyl)-propan und 1,1-Bis-(4-hydroxyphenyl)-3,3,5-trimethylcyclohexan, ganz besonders bevorzugt 2,2-Bis-(4-hydroxyphenyl)-propan.

Erfindungsgemäß einsetzbare aromatische Hydroxyverbindungen (II), die den Diarylcarbonaten (I) zugrundeliegen, sind beispielsweise: Phenol, o-, m-, p-Kresol, o-, m-, p-Ethyl-phenol und o-, m-, p-Chlor-phenol, bevorzugt Phenol und o-, m-, p-Kresol, besonders bevorzugt Phenol.

Bevorzugte Diarylcarbonate sind Diester von Phenol oder alkylsubstituierten Phenolen, z.B. Diphenylcarbonat oder z.B. Dikresylcarbonat, besonders bevorzugt Diphenylcarbonat (DPC).

Die Herstellung der Diarylcarbonate durch oxidative Carbonylierung erfolgt in bekannter Weise mit Hilfe eines Katalysatorsystems bestehend aus einer Base, einem quaternären Salz, einem Platinmetall oder einer Platinmetallverbindung und einem Cokatalysator.

Erfindungsgemäß zur Diarylcarbonatsynthese einsetzbare Basen sind tertiäre Amine, wie beispielsweise Trimethylamin, Triethylamin, Tripropylamin, Tributylamin, Tripentylamin, Alkalimetall-hydroxyverbindungen, wie Lithiumhydroxyd, Natriumhydroxyd, Kaliumhydroxyd, Rubidiumhydroxyd und Cäsiumhydroxyd, Alkalimetallsalze von aromatischen Hydroxyverbindungen der Formel (II), in der $R^1$ die oben angegebene Bedeutung hat. Ganz besonders bevorzugt wird ein Alkalimetallsalz der aromatischen Hydroxyverbindung (II) verwendet, die auch zum organischen Carbonat (I) umgesetzt werden soll. Diese Alkalimetallsalze können Lithium-, Natrium-, Kalium-, Rubidium- oder Cäsiumsalze sein. Bevorzugt werden Lithium-, Natrium-, und Kaliumphenolate, besonders bevorzugt Natriumphenolat eingesetzt. Das Alkalimetallphenolat kann dem Reaktionsgemisch als reine Verbindung in fester Form oder als Schmelze zugesetzt werden. Selbstverständlich können in das erfindungsgemäße Verfahren auch die Hydrate der Alkalimetallphenolate eingesetzt werden. Als Beispiel für ein solches Hydrat sei hier, ohne das erfindungsgemäße Verfahren einzuschränken, Natriumphenolat-trihydrat genannt. Die Menge an zugesetztem Wasser ist jedoch vorzugsweise so bemessen, daß pro Mol Base maximal 5 Mol Wasser eingesetzt werden. Höhere Wasserkonzentrationen führen meist zu schlechteren Umsätzen und Zersetzung gebildeter Carbonate. In einer weiteren Ausführungsform der Erfindung wird das Alkalimetallphenolat dem Reaktionsgemisch als Lösung, die 0,1 - 80 Gew.-%, bevorzugt 0,5 - 65 Gew.-%, besonders bevorzugt 1 - 50 Gew.-% des Alkalimetallphenolats enthält, zugesetzt. Als Lösungsmittel können hierbei sowohl Alkohole oder Phenole, wie beispielsweise das umzusetzende Phenol, als auch inerte Lösungsmittel verwendet werden. Als solche seien die weiter unten als Reaktionsmedien erwähnten genannt. Diese Lösungsmittel können allein oder in beliebiger Kombination miteinander eingesetzt werden. So besteht eine Ausführungsform des erfindungsgemäßen Verfahrens beispielsweise darin, daß man die Base in einer Phenolschmelze löst, die mit einem inerten Lösungsmittel verdünnt wurde. Bevorzugt wird die Base in der Schmelze einer aromatischen Hydroxyverbindung gelöst. Besonders bevorzugt wird die Base in einer Schmelze der aromatischen Hydroxyverbindung gelöst, die zum organischen Carbonat umgesetzt werden soll. Ganz besonders bevorzugt wird die Base, in Phenol gelöst, zugesetzt. Die Base wird in einer von der Stöchiometrie unabhängigen Menge zugesetzt. Das Verhältnis von Platinmetall, beispielsweise Palladium, zu Base wird vorzugsweise so gewählt, daß pro Grammatom Platinmetall, beispielsweise Palladium, 0,1 bis 5000, bevorzugt 3 bis 2000 besonders bevorzugt 9 bis 1000 Äquivalente Base eingesetzt werden. Das Platinmetall wird hierbei als Metall gerechnet, liegt aber unabhängig hiervon in metallischer oder gebundener Form in den verschiedenen bekannten Oxidationsstufen vor.

Das erfindungsgemäße Verfahren zur Diarylcarbonatherstellung wird vorzugsweise ohne Lösungsmittel durchgeführt. Selbstverständlich können auch inerte Lösungsmittel verwendet werden. Als Beispiele für Lösungsmittel seien Dimethylacetamid, N-Methylpyrrolidinon, Dioxan, t-Butanol, Cumylalkohol, Isoamylalkohol, Tetramethylharnstoff, Diethylenglykol, halogenierte Kohlenwasserstoffe, wie Chlorbenzol oder Dichlorbenzol, und Ether genannt.

Die erfindungsgemäß zur Diarylcarbonatherstellung geeigneten Platinmetall-Katalysatoren bestehen aus mindestens einem Metall der Gruppe VIII (CAS-Nomenklatur), vorzugsweise Palladium. Es kann bei dem erfindungsgemäßen Verfahren in verschiedener Form zugegeben werden. Palladium kann in metallischer Form oder bevorzugt in Form von Palladium-Verbindungen der Oxidationsstufen 0 und +2, wie beispielsweise Palladium(II)-acetylacetonat, -halogenide, -carboxylate von $C_2$-$C_6$-Carbonsäuren, -nitrat, -oxide oder Palladiumkomplexen, die beispielsweise Kohlenmonoxid, Olefine, Amine, Phosphine und Halogenide enthalten können, eingesetzt werden. Besonders bevorzugt sind Palladiumbromid und Palladium-acetylacetonat.

Die Menge an Platinmetall-Katalysator ist im erfindungsgemäßen Verfahren nicht beschränkt. Bevorzugt wird so viel Katalysator zugesetzt, daß die Konzentration des Metalls im Reaktionsansatz 1 - 3000 ppm beträgt, besonders bevorzugt sind Konzentrationen von 5 - 500 ppm.

Als Cokatalysator für das erfindungsgemäße Verfahren wird ein Metall der Gruppen III B, IV B, V B, I B, II B, VI B, VII B des Periodensystems der Elemente (CAS-Nomenklatur) oder der Eisengruppe in Form von Verbindung eingesetzt, wobei das Metall in verschiedenen Oxidationsstufen eingesetzt werden kann. Ohne das erfindungsgemäße Verfahren einzuschränken, seien Verbindungen von Mangan(II), Mangan(III), Kupfer(I), Kupfer(II), Kobalt(II), Kobalt(III), Vanadium(III) und Vanadium(IV) genannt. Die Metalle können beispielsweise als Halogenide, Oxide, Carboxylate von $C_2$-$C_6$-Carbonsäuren, Diketonate oder Nitrate sowie als Komplexverbindungen, die beispielsweise Kohlenmonoxid, Olefine, Amine, Phosphine und Halogenide enthalten können, eingesetzt werden. Bevorzugt werden Manganverbindungen im erfindungsgemäßen Verfahren verwendet, besonders bevorzugt Mangan(II)Verbindungen oder -komplexe, ganz besonders bevorzugt Mangan(II)acetylacetonat.

Der Cokatalysator wird in einer Menge zugesetzt, daß seine Konzentration im Bereich von 0,0001 bis 20 Gew.-% des Reaktionsgemisches liegt, bevorzugt ist der Konzentrationsbereich von 0,005 bis 5 Gew.-%, besonders bevorzugt 0,01 bis 2 Gew.-%.

Bei den im Rahmen der vorliegenden Erfindung eingesetzten quaternären Salzen kann es sich beispielsweise um mit organischen Resten substituierte Ammonium-, Phosphonium- oder Sulfoniumsalze handeln. Geeignet für den Einsatz in das erfindungsgemäße Verfahren sind Ammonium-, Phosphonium- und Sulfoniumsalze, die als organische Reste $C_6$- bis $C_{10}$-Aryl-, $C_7$- bis $C_{12}$-Aralkyl- und/oder $C_1$- bis $C_{20}$-Alkyl-Reste und als Anion ein Halogenid, Tetrafluoroborat oder Hexafluorophosphat tragen. Die organischen Reste der quaternären Salze können gleichartig oder verschiedenartig aus den genannten Gruppen sein. Bevorzugt werden in das erfindungsgemäße Verfahren Ammoniumsalze, die als organische Reste $C_6$- bis $C_{10}$-Aryl-, $C_7$- bis $C_{12}$-Aralkyl- und/oder $C_1$- bis $C_{20}$-Alkyl-Reste und als Anion ein Halogenid tragen, besonders bevorzugt ist Tetrabutylammoniumbromid. Die Menge eines solchen quaternären Salzes kann beispielsweise 0,1 - 20 Gew.-%, bezogen auf das Gewicht des Reaktionsgemisches, betragen. Vorzugsweise beträgt diese Menge 0,5 - 15 Gew-%, besonders bevorzugt 1 - 5 Gew.-%.

Das erfindungsgemäße Verfahren wird, vorzugsweise ohne Lösungsmittel, bei 30 bis 200°C, bevorzugt bei 30 bis 150°C, besonders bevorzugt bei 40 bis 120°C bei einem Druck von 1 bis 200 bar, bevorzugt von 2 bis 100 bar, besonders bevorzugt bei 5 bis 50 bar durchgeführt.

Die Zusammensetzung der Reaktionsgase Kohlenmonoxid und Sauerstoff kann in weiten Konzentrationsgrenzen variiert werden, es wird jedoch zweckmäßigerweise ein CO:$O_2$-Molverhältnis (normiert auf CO) von 1:(0,001-1,0) bevorzugt 1:(0,01-0,5) und besonders bevorzugt von 1:(0,02-0,3) eingestellt. Der Sauerstoffpartialdruck ist bei diesen Molverhältnissen groß genug, um hohe Raum-Zeit-Ausbeuten erreichen zu können und gleichzeitig keine explosionsfähigen Kohlenmonoxid/Sauerstoff-Gasgemische bilden zu können. Die Reaktionsgase unterliegen keinen besonderen Reinheitsanforderungen, so kann Synthesegas als CO-Quelle und Luft als $O_2$-Träger dienen, es ist jedoch darauf zu achten, daß keine Katalysatorgifte, wie beispielsweise Schwefel oder dessen Verbindungen, eingetragen werden. In der bevorzugten Ausführungsform des erfindungsgemäßen Verfahrens werden reines CO und reiner Sauerstoff verwendet.

Im erfindungsgemäßen Verfahren wird das in den Edukten enthaltene Wasser und das kontinuierlich durch die Reaktion entstehende Wasser durch überschüssiges Reaktionsgas ausgestrippt. Das Reaktionsgas, bestehend aus Kohlenmonoxid, Sauerstoff und einem Inertgas, wie Stickstoff, Methan, Neon oder Argon, bevorzugt Stickstoff, wird hierzu in einer Menge von 1 - 100000 Nl pro Liter Reaktionslösung, bevorzugt 5 - 50000 Nl pro Liter Reaktionslösung und besonders bevorzugt 10 - 10000 Nl pro Liter Reaktionslösung und Stunde eingeleitet.

Die als Rückstrom von der Polycarbonatanlage vorliegenden Menge an aromatischer Hydroxyverbindung wird zu 10 bis 100 %, bevorzugt 15 bis 100 % und besonders bevorzugt 20 bis 100 % in den Diarylcarbonatreaktor eingespeist. In einer weiteren Ausführungsform können restliche Mengen an aromatischer Hydroxyverbindung aus der Schmelzpolycarbonatherstellung, also 90 bis 0 %, bevorzugt 85 bis 0 %, besonders bevorzugt 80 bis 0 %, und nur für den Fall, daß die herausgespaltene aromatische Hydroxyverbindung auch dem Bisphenol zugrundeliegt, auch in der Herstellung des Bisphenols eingesetzt werden, wobei die Grenze 0 % (Null) den Fall anzeigt, bei dem keine aromatische Hydroxyverbindung aus dem Rückstrom von der Polycarbonatanlage zur Herstellung des Bisphenols eingesetzt wird.

In idealisierter Form können diese Zusammenhänge am Beispiel des Bishenol A-Poly-/Oligocarbonats wie folgt dargestellt werden:

(1)

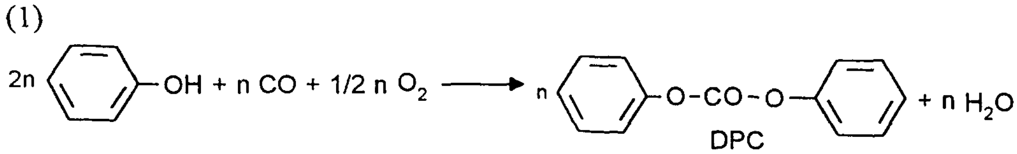

DPC

(2)

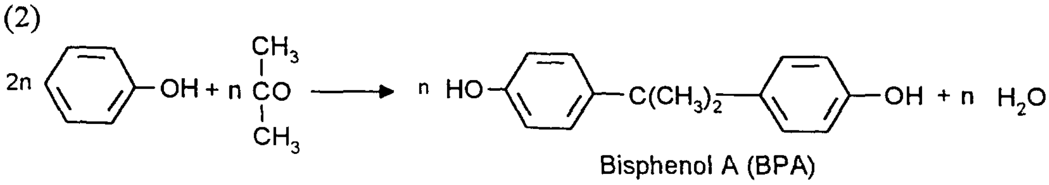

Bisphenol A (BPA)

(3)

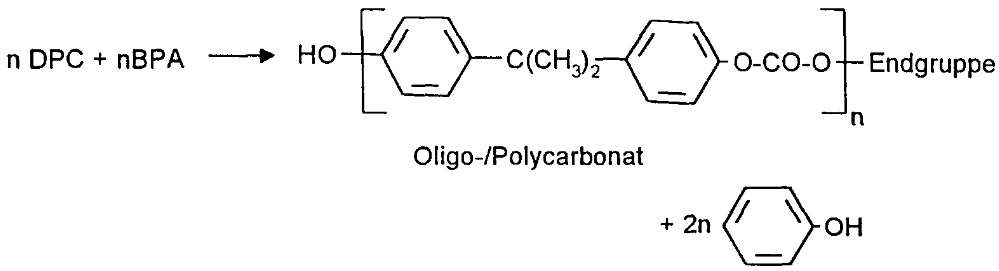

Oligo-/Polycarbonat

Von den 4n Mol eingesetzten Phenols in Gleichungen (1) und (2) werden demnach in der Schmelzpolykondensation nach Gleichung (3) 2n Mol wieder herausgespalten und können in Gleichung (1) oder in Gleichungen (1) und (2) zurückgeführt werden.

Die Isolierung und Reinigung des nach dem erfindungsgemäßen Verfahrens hergestellten Diarylcarbonats kann nach dem bekannten Stand der Technik durch Destillation, Extraktion und/oder Kristallisation erfolgen.

Das so hergestellte Diarylcarbonat wird nun direkt zur Umesterung (Schmelzpolykondensation) mit einer aromatischen Dihydroxyverbindung (Bisphenol) eingesetzt. Bezogen auf 1 mol Bisphenol werden die Diarylcarbonate in einer Menge von 1,01 bis 1,30 mol, bevorzugt in 1,02 bis 1,15 mol eingesetzt. Durch das molare Verhältnis wird der Polykondensationsgrad und damit das Molekulargewicht eingestellt; man gelangt so zu Oligocarbonaten oder thermoplastischen Polycarbonaten der unten angegebenen Molmassen. Die Herstellung der Polycarbonate kann einstufig oder über Oligocarbonate in 2-4 Stufen erfolgen. Oligocarbonate sind so günstig lagerfähige Vorstufen für Polycarbonate.

Die Polycarbonate können durch den Einsatz geringer Mengen Verzweiger bewußt und kontrolliert verzweigt werden. Einige geeignete Verzweiger sind: Phloroglucin, 4,6-Dimethyl-2,4,6-tri-(4-hydroxyphenyl)-hepten-2, 4,6-Dimethyl-2,4,6-tri-(4-hydroxyphenyl)-heptan, 1,3,5-Tri-(4-hydroxyphenyl)-benzol, 1,1,1-Tri-(4-hydroxy-phenyl)-ethan, Tri-(4-hydroxyphenyl)-phenylmethan, 2,2-Bis-[4,4-bis-(4-hydroxyphenyl)-cyclohexyl]-propan, 2,4-Bis-(4-hydroxyphenyl-isopropyl)-phenol, 2,6-Bis-(2-hydroxy-5'-methylbenzyl)-4-methylphenol, 2-(4-Hydroxyphenyl)-2-(2,4-dihydroxyphenyl)-propan, Hexa-(4-(4-hydroxyphenyl-isopropyl)-phenyl)-orthoterephthalsäureester, Tetra-(4-hydroxyphenyl)-methan, Tetra-(4-(4-hydroxyphenyl-isopropyl)-phenoxy)-methan, 1,4-Bis-(4',4"-dihydroxytriphenyl)-methyl)-benzol und insbesondere a,a',a"-Tris-(4-hydroxyphenyl)-1,3,5-triisopropylbenzol. Weitere mögliche Verzweiger sind 2,4-Dihydroxybenzoesäure, Trimesinsäure, Cyanurchlorid und 3,3-Bis-(3-methyl-4-hydroxyphenyl)-2-oxo-2,3-dihydroindol. Die gegebenenfalls mitzuverwendenden Mengen von 0,05 bis 2 Mol% an Verzweigern, bezogen auf eingesetzte Bisphenole, können mit den Bisphenolen zusammen eingesetzt werden.

Es ist vorteilhaft, wenn die Reaktionskomponenten für die Herstellung von Oligocarbonaten oder für den ersten Schritt einer mehrstufigen Herstellung von Polycarbonaten, also die Bisphenole und die Diarylcarbonate frei von Alkali- und Erdalkaliionen sind, wobei Mengen von kleiner 0,1 ppm an Alkali- und Erdalkaliionen toleriert werden können. Derart reine Bisphenole und Diarylcarbonate sind beispielsweise erhältlich, indem man diese Stoffe umkristallisiert, wäscht oder destilliert. Erfindungsgemäß soll der Gehalt an Alkali- und Erdalkalimetallionen sowohl im Bisphenol als auch im Diarylcarbonat unterhalb von 0,1 ppm liegen.

Die Umesterungsreaktion des Bisphenols und des Diarylcarbonats in der Schmelze wird bevorzugt in zwei Stufen durchgeführt. In der ersten Stufe findet das Aufschmelzen des Bisphenols und des Diarylcarbonats bei Temperaturen von 80 - 250°C, bevorzugt 100 - 230°C, besonders bevorzugt 120 - 190°C unter normalem Druck in 0,01 - 5 Stunden, bevorzugt 0,25 - 3 Stunden statt. Nach Zugabe eines Katalysators wird durch Anlegen von Vakuum (bis herunter zu 2 mm Hg) und Erhöhung der Temperatur (auf bis zu 260°C) durch Abdestillieren der aromatischen Hydroxyverbindung (II) das Oligocarbonat aus dem Bisphenol und dem Diarylcarbonat hergestellt. Das so hergestellte Oligocarbonat hat eine mittlere Gewichtsmolmasse $\overline{M}_w$ (ermittelt durch Messung der rel. Lösungsviskosität in Dichlormethan oder in Mischungen gleicher Gewichtsmengen Phenol/o-Dichlorbenzol, geeicht durch Lichtstreuung) im Bereich von 2000 bis 18 000, bevorzugt von 4 000 bis 15 000. Hierbei wird die Hauptmenge (> 80 %) an aromatischer Hydroxyverbindung (II), die dem Diarylcarbonat (I) zugrundeliegt, wiedergewonnen und im Rahmen des oben genannten Bereichs zur erneuten Herstellung von Diarylcarbonat in den Prozeß zurückgeführt.

In der zweiten Stufe wird bei der Schmelzpolykondensation durch weiteres Erhöhen der Temperatur auf 250 - 320°C, bevorzugt 270 - 295°C und einem Druck von < 2 mm Hg das Polycarbonat hergestellt. Hierbei wird der Rest an aromatischer Hydroxyverbindung (II), die dem Diarylcarbonat (I) zugrundeliegt, gewonnen. Es treten geringe Verluste an (II) von < 5 %, bevorzugt von < 2 %, besonders bevorzugt von < 1 % auf, verursacht durch Endgruppen im Polycarbonat und restlichem (II) im Polycarbonat. Diese Verluste müssen durch entsprechende Mengen an (II) für die Herstellung des Diarylcarbonats ausgeglichen werden. Zur Unterdrückung einer Anhäufung von Verunreinigungen kann dem recyclisierten (II) ein Purge-Strom entnommen werden, bevor es zur Herstellung von Diarylcarbonat wieder eingesetzt wird.

Katalysatoren für die Umesterung eines Diarylcarbonats mit einem Bisphenol im erfindungsgemäßen Sinne sind alle anorganischen oder organischen basischen Verbindungen, beispielsweise: Lithium-, Natrium-, Kalium-, Cäsium-, Calcium-, Barium-, Magnesium-, -hydroxide, -carbonate, -halogenide, -phenolate, -diphenolate, -fluoride, -acetate, -phosphate, -hydrogenphosphate oder -boranate, Stickstoff- und Phosphorbasen wie beispielsweise Tetramethylammoniumhydroxid, Tetramethylammoniumacetat, Tetramethylammoniumfluorid, Tetramethylammoniumtetraphenylboranat, Tetraphenylphosphoniumfluorid, Tetraphenylphosphoniumtetraphenylboranat, Dimethyldiphenylammoniumhydoxid, Tetraethylammoniumhydroxid, DBU (Diaza-bicyclouncecan), DBN (Diaza-bicyclononan) oder Guanidinsysteme wie beispielsweise das 1,5,7-Triazabicyclo-[4,4,0]-dec-5-en, 7-Phenyl-1,5,7-triazabicyclo-[4,4,0]-dec-5-en, 7-Methyl-1,5,7-triazabicyclo-[4,4,0]-dec-5-en, 7,7'-Hexyliden-di-1,5,7-triazabicyclo-[4,4,0]-dec-5-en, 7,7'-Decyliden-di-1,5,7-triazabicyclo-[4,4,0]-dec-5-en oder 7,7'-Dodecyliden-di-1,5,7-triazabicyclo-[4,4,0]-dec-5-en oder Phosphazene wie beispielsweise die Phosphazen-Base $P_1$-t-Oct = tert-Octyl-imino-tris-(dimethylamino)-phosphoran, Phosphazen-Base $P_1$-t-Butyl = tert.-Butyl-imino-tris-(dimethylamino)-phosphoran oder BEMP = 2-tert.-Butylimino-2-diethylamino-1,3-dimethyl-perhydro-1,3-diaza-2-phosphorin.

Diese Katalysatoren werden in Mengen von $10^{-2}$ bis $10^{-8}$ mol pro mol Bisphenol eingesetzt. Die Katalysatoren können auch in Kombination (zwei oder mehrere) miteinander eingesetzt werden.

Beim Einsatz von Alkali-/ Erdalkalimetallkatalysatoren kann es vorteilhaft sein, sie zu einem späteren Zeitpunkt (z. B. nach der Oligocarbonatsynthese bei der Polykondensation in der zweiten Stufe) zuzusetzen. Die Zugabe des Alkali-/ Erdalkalimetallkatalysators kann z. B. als Feststoff oder als Lösung in Wasser, Phenol, Oligocarbonat oder Polycarbonat erfolgen. Die Mitverwendung von Alkali- bzw. Erdalkalimetallkatalysatoren widerspricht nicht der vorstehend erwähnten Forderung nach Reinheit der Reaktionspartner.

Die Reaktion des Bisphenols und des Diarylcarbonats zum Polycarbonat kann im erfindungsgemäßen Sinne diskontinuierlich oder bevorzugt kontinuierlich durchgeführt werden, beispielsweise in Rührkesseln, Dünnschichtverdampfern, Fallfilmverdampfern, Rührkesselkaskaden, Extrudern, Knetern, einfachen Scheibenreaktoren und Hochviskosscheibenreaktoren.

Die aromatischen Polycarbonate des erfindungsgemäßen Verfahrens haben mittlere Gewichtsmolmassen $\overline{M}_w$ von 18000 bis 80000 vorzugsweise 19000 - 50000, ermittelt durch Messung der rel. Lösungsviskosität in Dichlormethan oder in Mischungen gleicher Gewichtsmengen Phenol/o-Dichlorbenzol, geeicht durch Lichtstreuung.

Das rohe (II), das beim Umesterungsprozeß isoliert wird, kann je nach Umesterungsbedingungen und Destillationsbedingungen u.a. mit Diarylcarbonat, dem Bisphenol, Salicylsäure, Salicylsäurephenylester, Isopropenylphenol, Phenoxybenzoesäurephenylester, Xanthon oder dem Hydroxymonoarylcarbonat verunreinigt sein. Eine Reinigung ist nicht erforderlich. Die Reinheit ist ausreichend für die erfindungsgemäße Recyclisierung zur Herstellung von Diarylcarbonat.

## Beispiel

Die beigefügte Figur 1 zeigt ein Blockschema der zur Durchführung des erfindungsgemäßen Verfahrens zur Herstellung eines Diarylcarbonats bzw. des erfindungsgemäßen Verfahrens zur Herstellung von Polycarbonaten eingesetzten flexiblen Versuchsanlage. Als Apparate zeigt die Figur 1 den Reaktor A zur Herstellung von Diphenylcarbonat (DPC), die Einrichtung B zur Abtrennung des DPC aus dem aus A abfließenden rohen Reaktionsgemisch, wobei in B gegebenenfalls auch eine weitergehende Reinigung des DPC vorgenommen wurde, die Umesterungsreaktoren C und D für die erste und zweite Stufe der Oligomerisierung und den Umesterungsreaktor E zur Schmelzpolykondensation für den Fall der Durchführung in mehreren Stufen, hier beispielsweise als dritte Stufe.

In den Reaktor A wurden Kohlenmonoxid und Sauerstoff durch die Zuleitungen (1) bzw. (2) ($CO:O_2$-Molverhältnis (normiert auf CO) 1:0,035) als Feedgas eindosiert. Der Sauerstoffpartialdruck ist bei diesen Molverhältnissen groß genug, um hohe Raum-Zeit-Ausbeuten von ca. 150 g DPC/l.h erreichen zu können und gleichzeitig keine explosionsfähigen Kohlenmonoxid/Sauerstoff-Gasgemische bilden zu können. Durch die separate Dosierung von Kohlenmonoxid und Sauerstoff ist die optimale Sauerstoffkonzentration gewährleistet.

Durch die Zuleitung (3) erfolgte die Phenol-Einleitung in den Reaktor A, während durch Leitung (4) Ergänzungen von Phenol und Katalysatorsystem zugespeist wurden. Die Temperatur im Reaktor A betrug 80°C bei einem Gesamtdruck von 10 bar (abs.). Das bei der Reaktion gebildete Wasser wurde mit überschüssigem Feedgas durch die Abgasleitung (15) ausgestrippt und vom Feedgas abgetrennt. Das überschüssige Feedgas wurde - nach Entnahme eines Purgestromes (Leitung 19) zur Abtrennung von Inertgasen und Kohlendioxid - durch Leitung (16) in den Reaktor A zurückgeführt.

Das rohe, Diphenylcarbonat enthaltende Reaktionsgemisch wurde dem Reaktor A kontinuierlich durch Leitung (5) entnommen und der Einrichtung B zugeführt, wo die Abtrennung und Reinigung des gebildeten DPC erfolgte. Über Leitung (6) wurde die das Katalysatorsystem enthaltende Mutterlauge in den Reaktor A zurückgeführt. In B gewonnenes DPC wurde über Leitung (7) der ersten Stufe der Umesterung/Schmelzpolykondensation C zugeführt. Weiterhin wurde C ein Strom von 2,2-Bis-(4-hydroxyphenyl)-propan (Bisphenol A) durch Leitung (8) zugesetzt.

Die Umsetzung in C und D erfolgte in Gegenwart von $10^{-6}$ Mol Tetraphenylphosphonium-tetraphenylboranat pro Mol umgesetzten Bisphenols A als Umesterungskatalysator zunächst bei einer Temperatur von 180°C, die auf 220°C gesteigert wurde, wobei gleichzeitig der Druck auf 100 mbar reduziert wurde. Das herausgespaltene Phenol wurde über Leitung (9) in den Reaktor A zurückgeführt, das Oligocarbonat wurde durch Leitung (10) in die zweite Stufe der Umesterung/Schmelzpolykondensation D eingespeist. In D wurde bei einer Temperatur von 250°C und einem Druck von 5 mbar weiteres Phenol herausgespalten und über Leitung (11) in den Reaktor A zurückgeführt. Dem Umesterungsreaktor D wurde über Leitung (12) ein Oligocarbonat mit einem Molgewicht von ca. 5.000 g/mol entnommen und in die dritte Stufe (Polykondensationsreaktor E) geführt. In E wurden bei einer Temperatur von 290°C und einem Druck von 0,1 mbar in Gegenwart von $10^{-6}$ Mol NaOH pro Mol umgesetzten Bisphenols A letzte Reste von Phenol herausgespalten und über Leitung (17) zusammen mit dem in C und D herausgespaltenen Phenol nach Entnahme eines Purge-Stromes (13) über die Sammelleitung (14) in den Reaktor A zurückgeführt. Dem Polykondensationsreaktor E wurde Polycarbonat als Verfahrensprodukt (Leitung 18) entnommen, das ein Molgewicht von ca. 25.000 g/mol aufweist. Das Polycarbonat war farbhell und lösungsmittelfrei. Die Versuchsanlage wurde nach 21 Tagen kontinuierlichen Betriebs abgestellt, ohne daß Anzeichen von Qualitätseinbußen oder ein Absinken des erzielten Molgewichts erkennbar waren.

## Patentansprüche

1. Verfahren zur Herstellung von Polycarbonaten durch Schmelzpolykondensation eines Diarylcarbonats mit einem Bisphenol unter Herausspalten der dem Diarylcarbonat zugrundeliegenden aromatischen Hydroxyverbindung, **gekennzeichnet durch** die Schritte

   a) Herstellung eines Diarylcarbonats der Formel

   $$R^1\text{-O-CO-O-}R^1 \qquad (I)$$

   **durch** oxydative Carbonylierung der zugrundeliegenden aromatischen Hydroxyverbindung der Formel

   $$R^1\text{-OH} \qquad (II),$$

   wobei in den Formeln

   $R^1$ Phenyl, Methyl-phenyl, Ethyl-phenyl oder Chlor-phenyl bedeutet,

   mit Kohlenmonoxid und Sauerstoff in Gegenwart eines Platinmetall-Katalysators, eines Cokatalysators, eines quaternären Salzes und einer Base,

   b) Abtrennung und Reinigung des Diarylcarbonats,

   c) Umesterung des Diarylcarbonats mit einem Bisphenol der Formel

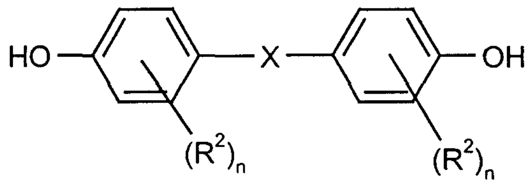

(III),

worin

X eine Einfachbindung, -S-, $-SO_2-$, -CO-, -O-, $C_1$-$C_8$-Alkyliden oder $C_5$-$C_9$-Cycloalkyliden,
$R^2$ Methyl, Cl oder Br und
n eine der Zahlen 0, 1 oder 2 bedeuten,

in einer Schmelzpolykondensation unter Bildung des korrespondierenden Oligo- oder Polycarbonats und Herausspalten der aromatischen Hydroxyverbindung (II),

d) Abtrennung der aromatischen Hydroxyverbindung (II) vom Oligo- bzw. Polycarbonat und

e) Rückführung von (II) in den Schritt a) ohne weitere Reinigung.

2. Verfahren nach Anspruch 1, **dadurch gekennzeichnet, dass** als Bisphenol 2,2-Bis-(4-hydroxyphenyl)-propan oder 1,1-Bis-(4-hydroxyphenyl)-3,3,5-trimethylcyclohexan eingesetzt wird.

3. Verfahren nach Anspruch 1, **dadurch gekennzeichnet, dass** als aromatische Hydroxyverbindung Phenol oder o-, m- oder p-Kresol eingesetzt wird.

4. Verfahren nach Anspruch 1, **dadurch gekennzeichnet, dass** als Base tertiäre Amine, Alkalimetallhydroxide oder Alkalimetallsalze aromatischer Hydroxyverbindungen (II) eingesetzt werden.

5. Verfahren nach Anspruch 1, **dadurch gekennzeichnet, dass** als Platinmetall-Katalysatoren Palladium in metallischer Form oder in Form einer Pd-Verbindung der Oxidationsstufe 0 oder +2 eingesetzt wird.

6. Verfahren nach Anspruch 1, **dadurch gekennzeichnet, dass** als Cokatalysator ein Metall der Gruppen III B, IV B, V B, I B, II B, VI B, VII B oder der Eisengruppe in Form einer Verbindung eingesetzt wird.

7. Verfahren nach Anspruch 1, **dadurch gekennzeichnet, dass** als quaternäres Salz ein mit organischen Resten substituiertes Ammonium-, Phosphonium- oder Sulfoniumsalz eingesetzt wird.

8. Verfahren nach Anspruch 1, **dadurch gekennzeichnet, dass** 10-100 % der bei der Schmelzpolykondensation herausgespaltenen aromatischen Hydroxyverbindung in die Herstellung des Diarylcarbonats zurückgeführt wird.

## Claims

1. A process for preparing polycarbonates by melt polycondensation of a diaryl carbonate with a bisphenol, with elimination of the parent aromatic hydroxy compound of the diaryl carbonate, **characterised by** the steps

a) preparation of a diaryl carbonate of the formula

$$R^1\text{-O-CO-O-}R^1 \quad (I)$$

by oxidative carbonylation of the parent aromatic hydroxy compound of the formula

$$R^1\text{-OH} \qquad \text{(II)},$$

wherein, in the formulae

$R^1$ represents phenyl, methylphenyl, ethylphenyl or chlorophenyl,

with carbon monoxide and oxygen in the presence of a platinum metal catalyst, a co-catalyst, a quaternary salt and a base,

b) separation and purification of the diaryl carbonate,

c) transesterification of the diaryl carbonate with a bisphenol of the formula

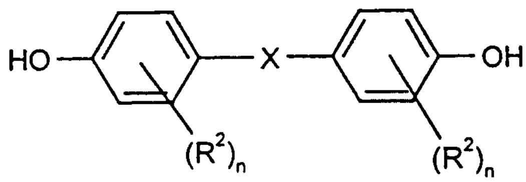

(III),

in which

X represents a single bond, -S-, $-SO_2-$, -CO-, -O-, $C_1$-$C_8$-alkylidenes or $C_5$-$C_9$-cycloalkylidenes,

$R^2$ represents methyl, Cl or Br and

n represents the numbers 0, 1 or 2,

in a melt polycondensation process with formation of the corresponding oligocarbonate or polycarbonate and elimination of the aromatic hydroxy compound (II),

d) separation of the aromatic hydroxy compound (II) from the oligocarbonate or polycarbonate and

e) the recycling of (II) to step a) without further purification.

2. A process according to Claim 1, **characterised in that** 2,2-bis-(4-hydroxyphenyl)-propane or 1,1-bis-(4-hydroxyphenyl)-3,3,5-trimethylcyclohexane is used as a bisphenol.

3. A process according to Claim 1, **characterised in that** phenol or o-, m- or p-cresol is used as an aromatic hydroxy compound.

4. A process according to Claim 1, **characterised in that** tertiary amines, alkali metal hydroxides or alkali metal salts of aromatic hydroxy compounds (II) are used as bases.

5. A process according to Claim 1, **characterised in that** palladium in the metallic form or in the form of a Pd compound in oxidation state 0 or +2 is used as a platinum metal catalyst.

6. A process according to Claim 1, **characterised in that** a metal from the groups IIIB, IVB, VB, IB, IIB, VIB, VIIB or the iron group, in the form of a compound, is used as co-catalyst.

7. A process according to Claim 1, **characterised in that** an ammonium, phosphonium or sulfonium salt substituted with organic groups is used as a quaternary salt.

8. A process according to Claim 1, **characterised in that** 10-100 % of the aromatic hydroxy compound eliminated during melt polycondensation is recycled to prepare the diaryl carbonate.

## Revendications

1. Procédé de préparation de polycarbonates par polycondensation à l'état fondu d'un carbonate de diaryle avec un bisphénol avec libération du composé hydroxylé aromatique à la base du carbonate de diaryle, qui est **caractérisé par** les étapes consistant à :

   a) préparer un carbonate de diaryle de formule :

   $$R^1 - O - CO - O - R^1 \qquad (I)$$

   par carbonylation oxydative du composé hydroxylé aromatique de base de formule (II):

   $$R^1 - OH \qquad (II)$$

   où dans les formules:

   $R^1$ représente les radicaux phényle, méthylphényle, éthylphényle ou chlorophényle,

   avec du monoxyde de carbone et de l'oxygène en présence d'un catalyseur de platinoïde métallique, d'un cocatalyseur, d'un sel quaternaire et d'une base ;
   b) séparer et purifier le carbonate de diaryle ;
   c) transestérifier le carbonate de diaryle avec un bisphénol de formule :

   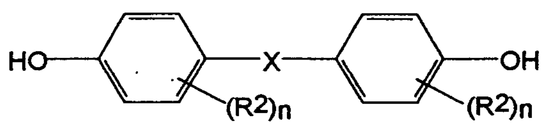

   (III)

   où :

   X représente une simple liaison, les radicaux -S-, $-SO_2-$, -CO-, -O-, alcoylidène en $C_1$-$C_8$ ou cycloalcoylidène en $C_5$-$C_9$ ;
   $R^2$ représente le radical méthyle, les atomes Cl ou Br, et
   n représente l'un des nombres 0, 1 ou 2,

   dans une polycondensation à l'état fondu, avec formation de l'oligo- ou polycarbonate correspondant et libération du composé hydroxylé aromatique (II) ;
   d) séparer le composé hydroxylé aromatique (II) de l'oligo- ou polycarbonate, et
   e) recycler (II) dans l'étape a) sans autre purification.

2. Procédé suivant la revendication 1, **caractérisé en ce que** l'on met en oeuvre comme bisphénol, le 2,2-bis(4-hydroxyphényl)propane ou le 1,1-bis(4-hydroxyphényl)-3,3,5-triméthylcyclohexane.

3. Procédé suivant la revendication 1, **caractérisé en ce que** l'on met en oeuvre comme composé hydroxylé aromatique, le phénol, le o-, le m- ou le p-crésol.

4. Procédé suivant la revendication 1, **caractérisé en ce que** l'on met en oeuvre comme base, des amines tertiaires, des hydroxydes de métal alcalin ou des sels de métal alcalin du composé hydroxylé aromatique (II).

5. Procédé suivant la revendication 1, **caractérisé en ce que** l'on met en oeuvre comme catalyseur de métal du groupe du platine, le palladium sous forme métallique ou sous forme d'un composé du Pd d'étage d'oxydation 0 ou +2

6. Procédé suivant la revendication 1, **caractérisé en ce que** l'on met en oeuvre comme cocatalyseur, un métal des

groupes IIIB, IVB, VB, IB, IIB, VIB, VIIB ou du groupe du fer sous forme d'un composé.

7. Procédé suivant la revendication 1, **caractérisé en ce que** l'on met en oeuvre comme sel quaternaire, un sel d'ammonium, de phosphonium ou de sulfonium, substitué par des restes organiques.

8. Procédé suivant la revendication 1, **caractérisé en ce que** 10-100% du composé hydroxylé aromatique libéré dans la polycondensation à l'état fondu sont recyclés dans la préparation du carbonate de diaryle.

Fig. 1